(19) **Europäisches Patentamt / European Patent Office / Office européen des brevets**

(11) **EP 1 598 005 B1**

(12) **EUROPÄISCHE PATENTSCHRIFT**

(45) Veröffentlichungstag und Bekanntmachung des Hinweises auf die Patenterteilung:
**18.03.2009 Patentblatt 2009/12**

(51) Int Cl.:
*A61B 5/029* (2006.01)    *A61B 5/0215* (2006.01)
*A61B 5/028* (2006.01)

(21) Anmeldenummer: **05102713.4**

(22) Anmeldetag: **07.04.2005**

(54) **Vorrichtung zur Ermittlung eines hämodynamischen Parameters**

Device for determining hemodynamic parameters

Dispositif pour déterminer des paramètres hémodynamiques

(84) Benannte Vertragsstaaten:
**DE ES FR GB IT**

(30) Priorität: **17.05.2004 DE 102004024334**

(43) Veröffentlichungstag der Anmeldung:
**23.11.2005 Patentblatt 2005/47**

(73) Patentinhaber: **Pulsion Medical Systems AG**
**81829 München (DE)**

(72) Erfinder:
• **Pfeiffer, Ulrich J.**
**81667, München (DE)**
• **Knoll, Reinhold**
**81543, München (DE)**
• **Regh, Stephan**
**81673, München (DE)**

(74) Vertreter: **Ettmayr, Andreas et al**
**Kehl & Ettmayr**
**Patentanwälte**
**Friedrich-Herschel-Strasse 9**
**81679 München (DE)**

(56) Entgegenhaltungen:
**EP-A- 1 236 435          DE-A1- 19 814 371**

• **DATABASE MEDLINE [Online] US NATIONAL LIBRARY OF MEDICINE (NLM), BETHESDA, MD, US; Juli 1995 (1995-07), IRLBECK M ET AL: "[Continuous measurement of cardiac output with pulse contour analysis]" XP008050494 Database accession no. NLM7661336 & DER ANAESTHESIST. JUL 1995, Bd. 44, Nr. 7, Juli 1995 (1995-07), Seiten 493-500, ISSN: 0003-2417**

**Beschreibung**

Die vorliegende Erfindung betrifft eine Vorrichtung zur Ermittlung eines hämodynamischen Parameters eines Patienten mittels Pulskonturanalyse.

Die Bestimmung hämodynamischer Parameter, insbesondere des Herzeitvolumens (Cardiac Output CO), mittels Pulskonturanalyse auf Basis eines nichtlinearen Windkesselmodells ist in DE 198 14 371 A1 sowie darin aufgeführter weiterführender Literatur ausführlich beschrieben. Die grundlegende Meßgröße für die Pulskonturanalyse ist ein dem Aortendruck näherungsweise entsprechender Druck, welcher beispielsweise mittels eines arteriellen Katheters in einer Beinarterie fortlaufend gemessen wird. Ein Pulskonturanalyse-System der Pulsion Medical Systems AG ist unter der Bezeichnung PiCCO kommerziell erhältlich.

Wesentliche Größen bei der Bestimmung hämodynamischer Parameter ausgehend von der Funktion P(t), d.h. dem zeitlichen Verlauf des dem Aortendruck näherungsweise entsprechenden Drucksignals, sind insbesondere der systemische Gefäßwiderstand (Systemic Vascular Resistance, SVR) sowie ferner auch die sogenannte Compliance (C). Ersterer wird anschaulich als Durchströmungswiderstand des Gefäßsystems des großen Kreislaufs verstanden, letztere als Nachgiebigkeit im Bereich der Aorta. In einem Ersatzschaltbild lassen sich diese Größen als Widerstand und Kapazität darstellen. Insbesondere bei älteren Ansätzen wird die Compliance mitunter vernachlässigt.

Eine Vorrichtung und ein Verfahren zur Bestimmung der Compliance ist in DE 198 14 371 A1 offenbart.

Bei herkömmlichen Implementierungen der Pulskonturanalyse gehen in die Bestimmung des systemischen Gefäßwiderstands und der Compliance (wenn diese nicht vernachlässigt wird) Kalibrierwerte ein, welche im Rahmen einer Kalibriermessung ermittelt, und danach nicht mehr geändert werden. Diese Kalibriermessung umfaßt die Bestimmung eines Kalibrierwerts des Herzeitvolumens mittels transpulmonaler Thermodilutionsmessung.

Für das mittels Pulskonturanalyse ermittelte Herzeitvolumen (Pulse Contour Cardiac Output PCCO), welches als Produkt aus Pulsfrequenz (Heart Rate, HR) und Schlagvolumen (Stroke Volume, SV) berechnet wird, wird von folgenden Beziehungen Gebrauch gemacht.

Das Schlagvolumen (SV) wird durch Integration über eine Pulsperiode oder über die Systole gemäß der Beziehung

$$SV \propto \int \left( \frac{p(t)}{SVR} + C(p) \cdot \frac{dp}{dt} \right) dt$$

berechnet (mit Zeit t, Druck p, systemischer Gefäßwiderstand SVR, Compliance C).

Dabei wird das Integral für jede Pulsperiode k mittels numerischer Integration ausgewertet, worin die Compliance in der Form

$$C(p) = \frac{CO_{TD}}{\left\langle \frac{dp}{dt} \right\rangle_{Cal}} \cdot \frac{1}{\frac{3}{MAP_{Cal}} p - 3 - \frac{1}{MAP_{Cal}^2} p^2}$$

eingesetzt wird (mit COTD:= mittels transpulmonaler Thermodilutionsmessung bestimmter Kalibrierwert des Herzeitvolumens, <dp/dt>Cal:= mittlere [negative] Steigung der Druckkurve in der Diastole bei der Kalibriermessung, Pd:= diastolischer Druck; MADCal:=mittlerer arterieller Druck bei der Kalibriermessung).

Die Parameter COTD, MADCal und <dp/dt>Cal werden nur im Rahmen der Kalibriermessung ermittelt und dann für alle Herzeitvolumen-Berechnungen verwendet.

Auch für den systemischen Gefäßwiderstand SVR wird ein gemäß der Beziehung

$$SVRCal = (MAP - CVP)Cal / COTD$$

ermittelter Kalibrierwert eingesetzt (mit MAP:= mittlerem arteriellen Druck [Mean Arterial Pressure], CVP:= zentralvenöser Druck [Central Venous Presseure]), welcher im Rahmen der Kalibriermessung bestimmt und dann für alle Herzeitvolumen-Berechnungen als Konstante verwendet wird.

Es hat sich gezeigt, daß die mittels Pulskonturanalyse ermittelten hämodynamischen Parameter mit zunehmender Dauer der Patientenüberwachung (ab der Kalibriermessung gerechnet mitunter an Qualität abnehmen. D.h. die Wahrscheinlichkeit, daß der pulskonturanalytisch gewonnene hämodynamische Parameter von den tatsächlichen physiologischen Gegebenheiten um mehr als ein bestimmtes vorgegebenes Maß abweicht, nimmt zu.

Um dem gegenzusteuern kann die Kalibriermessung mittels Thermodilution in geringeren Abständen wiederholt werden. Dies ist jedoch mit einigem Aufwand, insbesondere mit der Vornahme einer Bolus-Injektion verbunden, was zusätzlichen Streß für den überwachten Patienten bedeutet, sowie das involvierte medizinische Personal zeitlich beansprucht und somit dessen Verfügbarkeit für andere Aufgaben vermindert. Die Druckschrift EP 1 236 435 A1 offenbart, für die regelmäßigen Rekalibrierung mittels Thermodilution einen beheizbaren zentralvenösen Katheter einzusetzen. Dies hilft zwar Bolusinjektionen zu vermeiden, erhöht jedoch den apparativen Aufwand erheblich.

Vor dem Hintergrund des obengesagten liegt der vorliegenden Erfindung die Aufgabe zugrunde, eine Vorrichtung zur Ermittlung hämodynamischen Parameter mittels Pulskonturanalyse zu schaffen, bei deren Einsatz auf regelmäßige Wiederholung einer Kalibriermessung mittels Thermodilution verzichtet werden kann, und welche dennoch eine Meßdatenauswertung gleichbleibender Güte liefert.

Gemäß einem Aspekt der Erfindung wird diese Aufgabe mit einer Vorrichtung zur Ermittlung eines hämodynamischen Parameters eines Patienten mittels Pulskonturanalyse nach Anspruch 1 gelöst.

Bevorzugte Ausführungsformen können gemäß einem der Ansprüche 2-17 gestaltet sein.

Es erfolgt also gewissermaßen eine fortlaufende Neukalibrierung des systemischen Gefäßwiderstands, vorzugsweise auch der Compliance, mittels aus den kontinuierlich ermittelten Druckmeßdaten gewonnener Parameter.

Erfindungsgemäß kann die pulskonturanalytische Bestimmung des Herzzeitvolumens PCCO in ihren Grundzügen so erfolgen, wie oben beschrieben, wobei jedoch ein oder mehrere der Parameter, welche herkömmlicherweise im Rahmen der Kalibriermessung bestimmt und dann als Konstante behandelt würden, regelmäßig, vorzugsweise einmal für jede Pulsperiode, aus der Funktion p(t) neuberechnet werden.

Insbesondere für den systemischen Gefäßwiderstand SVR erfolgt hierfür zunächst eine fortlaufende Neuberechnung. Grundsätzlich ist die Größe SVR aus der Beziehung

$$SVR = (MAP\text{-}CVP)/CO$$

herleitbar. Für das Herzzeitvolumen CO einfach das pulskonturanalytisch gewonnene Herzeitvolumen PCCO einzusetzen, führt jedoch zu einer Zirkelbeziehung. Dies läßt sich grundsätzlich mittels eines Iterationsprozesses lösen, was im Falle von drastischen Veränderungen des systemischen Gefäßwiderstands SVR jedoch zu Instabilitäten führen kann. Daher werden vorteilhafterweise zusätzliche oder alternative Kriterien für eine Änderung des systemischen Gefäßwiderstands SVR implementiert.

Geeignete Parameter, welche zur Berücksichtigung von Änderungen im systemischen Gefäßwiderstand SVR herangezogen werden können, sind insbesondere:

- der systolische Druck Ps,
- der mittlere arterielle Druck MAP,
- der Pulsdruck (Pulse Pressure) PP = (Ps-Pd),
- die Differenz aus dem Druck am Übergang zwischen Systole und Diastole und dem diastolischen Druck (Pn-Pd),
- mittlere Steigung der Druckkurve in der Diastole <dp/dt>.

Die diastolische Phase der Pulsperiode kann vorteilhafterweise durch exponentielles Abfallen angenähert werden:

$$p(t) - \textit{offset} = \textit{const.} \cdot \exp(-t/\tau)$$

wobei *offset* den Abszissenwert beim Beginn des exponentiellen Abfalls bezeichnet, $\exp(-t/\tau)$ eine Exponentialfunktion mit dem Argument $(-t/\tau)$ ist, und die Zeitkonstante $\tau$ aus der Druckkurve gemäß folgender Beziehung gewonnen werden kann:

$$\tau = [p(t)\text{-}\textit{offset}] / (dp/dt)$$

Für das vereinfachte Ersatzschaltbild einer Parallelschaltung aus Kapazität (Compliance) und Widerstand (systemischer Gefäßwiderstand) gilt:

$$\tau = SVR \cdot C(p).$$

Auch wenn diese Beziehungen grundsätzlich ausreichend sind, können die Ergebnisse durch Verwendung empirischer Beziehungen als Funktion der obigen Parameter verbessert werden, insbesondere durch die vorzugsweise angewandte Beziehung

$$SVR = SVRcal \cdot (\tau / \tau cal)^a \cdot (Pd / Pdcal)^b$$

worin SVRcal ein Kalibrierwert des systemischen Widerstands, $\tau$cal ein Kalibrierwert der Zeitkonstante $\tau$, Pd der diastolische arterielle Druck, Pdcal ein Kalibrierwert des diastolischen arterielle Drucks, und a, b empirisch bestimmte oder abgeschätzte Exponenten sind. Die Kalibrierwerte SVRcal, $\tau$cal und Pdcal können im Rahmen einer Kalibriermessung einschließlich Thermodilutionsmessung bestimmt sein. Die Parameterwahl a=0,3 und b=1 ergibt eine gute Anpassung, unter Umständen kann jedoch durch leichte Änderung dieser Parameter eine verbesserte Anpassung erzielt werden.

Auch der (arterielle) diastolische Druck Pd und der Druck am Übergang zwischen Systole und Diastole Pn werden vorzugsweise regelmäßig neu ermittelt, sofern diese für die weitergehende Berechnung hämodynamischer Parameter benötigt werden. Besonders vorteilhaft ist es dabei, verbesserte Wege zur Bestimmung des Übergangs zwischen Systole und Diastole, des sogenannten "Diachrotic Notch", sowie zur Bestimmung des diastolischen Drucks zu implementieren.

Unter Verwendung geeigneter Glättungsalgorithmen werden die erste (y' = dP/dt) und die zweite Ableitung (y" = $d^2p/dt^2$) der Funktion p(t) bestimmt. Aus diesen wird eine Indikationsfunktion berechnet, welche ein Maß für die lokale Krümmung der Funktion p(t) darstellt. Besonders gut geeignet ist die Krümmungsfunktion

$$K = y"/(1+y'^2)^{3/2}.$$

Diese kann als Kehrwert eines lokalen Krümmungsradius aufgefaßt werden. Vorzugsweise ist eine Achsanpassung für die Funktion p(t) vorgesehen dergestalt, daß ein aus empirisch erhobenen Daten gewonnener typischer Verlauf einer arteriellen Druckfunktion am Übergang zwischen Systole und Diastole annähernd die Gestalt eines Kreisbogens besitzt.

Innerhalb des Bereichs der Funktion p(t), in welchem diese Werte von 90 % bis 10 %, vorzugsweise 75 % bis 10 % ihres Maximalwerts innerhalb der aktuellen Pulsperiode annimmt, wird die Lage des Maximums der Krümmungsfunktion K bestimmt. Der entsprechende Zeitpunkt wird gegebenenfalls noch unter Berücksichtigung von Verzögerungsgliedern im Meßaufbau, beispielsweise Filtern, korrigiert. Liegt das Maximum der Krümmungsfunktion K (ggf. nach dieser Korrektur) innerhalb von 70 % der Dauer der aktuellen Pulsperiode (oder der Dauer einer vorhergehenden Pulsperiode, falls die Berechnung in Echtzeit vor Ende der aktuellen Pulsperiode ausgeführt wird), so wird der Ort des (ggf. korrigierten) Maximums der Krümmungsfunktion K als Zeitpunkt des Übergangs zwischen Systole und Diastole aufgefaßt. Andernfalls wird der Übergang zwischen Systole und Diastole auf 70 % der Dauer der aktuellen Pulsperiode (oder der Dauer einer vorhergehenden Pulsperiode, falls die Berechnung in Echtzeit vor Ende der aktuellen Pulsperiode ausgeführt wird) festgesetzt. Optional kann noch eine zusätzliche Plausibilitätskontrolle unter Berücksichtigung von Pulsdauer, Ausstoßzeit etc. vorgesehen sein.

Alternativ kann auf die Bestimmung der Krümmungsfunktion verzichtet werden, und anstelle des Maximums der Krümmungsfunktion K das Maximum der zweiten Ableitung y" der Funktion p(t), ggf. nach entsprechender Korrektur, als Zeitpunkt des Übergangs zwischen Systole und Diastole aufgefaßt werden.

Wie oben erwähnt, wird bei der Bestimmung des Schlagvolumens häufig nur über Druckwerte der Systole integriert. Auch um die Genauigkeit der Schlagvolumenbestimmung zu erhöhen, ist daher vorzugsweise obige verbesserte Bestimmung des Übergangs zwischen Systole und Diastole vorgesehen.

Bei der Ermittlung des diastolischen Drucks Pd hat sich der folgende Ansatz als besonders vorteilhaft erwiesen, welcher den Einfluß der limitierten Meßfrequenz berücksichtigt, d.h. der Frequenz, mit welcher das den arteriellen Druck ermittelnde Druckmeßsystem anspricht (und entsprechend als Tiefpaßfilter fungiert):

Ausgehend vom tiefsten gemessenen Druck wird eine lineare Regression geeigneter Länge vorgenommen.

Als geeignete Länge können vorteilhafterweise etwa 100 Millisekunden oder das doppelte des Kehrwerts der Meßfrequenz angenommen werden. Ist die Länge der Diastole kürzer als das doppelte dieser Länge, so wird der tiefste gemessene Druck als diastolischer Druck Pd angenommen. Andernfalls wird der Beginn der Regression um eine geeignete Strecke, vorzugsweise die Hälfte der Länge der Regression, in Richtung des systolischen Peaks verschoben und in die Gegenrichtung extrapoliert. Der Schnittpunkt der Extrapolation mit der extrapolierten linearen Regression der Druckkurve im Bereich ihrer maximalen Steigung gibt dann den diastolischen Druck vor. Für die Ermittlung des Bereichs der maximalen Steigung werden vorzugsweise nur Datenpunkte innerhalb von 20% bis 80% der Pulsamplitude (höchster gemessener Druck minus niedrigstem gemessenen Druck der Pulsperiode) berücksichtigt.

In der eingangs aufgeführten Berechnungsformel für die Compliance kann der Wert COTD durch den Ausdruck

$$(MAP-CVP)/SVR$$

ersetzt werden, wobei die Differenz zwischen mittlerem arteriellen Druck MAP und zentralvenösem Druck CVP regelmäßig neu bestimmt wird. Dabei können entweder die Werte der aktuellen bzw. vorangehenden Pulsperiode verwendet werden, oder aber gemittelte Werte über einige (beispielsweise zwischen 10 und 50) vorangehende Pulsperioden bzw. einige (beispielsweise ungefähr 30) Sekunden. Auch die mittlere Steigung der Druckkurve in der Diastole <dp/dt> wird vorzugsweise regelmäßig neu bestimmt.

Zu besseren Ergebnissen bei der Ermittlung der Compliance führt vorzugsweise eine Korrekturfunktion der Form

$$f(p)= 1 \ / \ [k1 \cdot \ p \ / \ MAP - k2 - k3 \cdot \ (p \ / \ MAP)^2]$$

mit empirirsch bestimmten oder abgeschätzten Koeffizienten k1, k2, k3. Zu einer geeigneten Anpssung führt die Wahl der Koeffizienten k1=9/5, k2=17/25 und k3=9/2. Daneben können andere geeignete Anpassungen existieren.

Für die Compliance ergibt sich also vorzugsweise

$$C(p)=(MAP-CVP)/(SVR \cdot <dp/dt>) \cdot \ \ f(MAP,p)$$

mit

$$f(MAP,p) = 1/[1,8 \cdot \ p/MAP - 0,68 - 4,5 \cdot \ (p \ /MAP)^2 \ ]$$

wobei die Parameter MAP, SVR und <dp/dt>, ggf. auch CVP regelmäßig neubestimmt werden.

Grundsätzlich erfindungsgemäß, wenn auch nicht bevorzugt, sind jedoch auch Ausführungen, bei welchen die Parameter der Compliance-Funktion nicht regelmäßig neuberechnet werden, oder die Compliance vernachlässigt wird.

Nachfolgend wird ein Beispiel einer besonders bevorzugten Ausführungsform der Erfindung anhand der zugehörigen, rein schematisch aufzufassenden Zeichnung näher beschrieben.

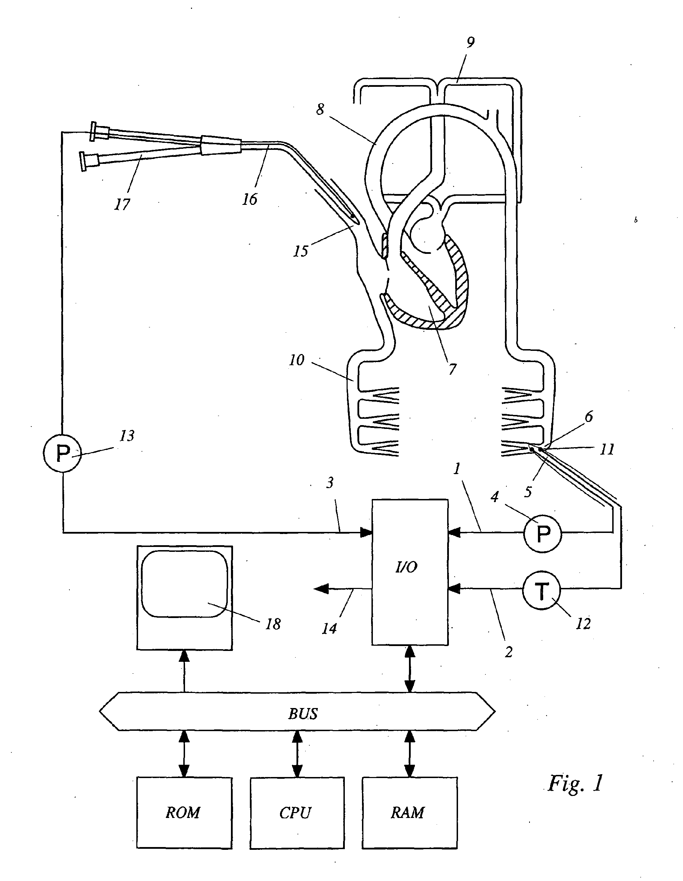

Fig. 1 zeigt dabei ein stark vereinfachtes Blockschaltbild einer erfindungsgemäßen Vorrichtung sowie einen skizzierten Ausschnitt des Gefäßsystems eines Patienten.

Die Vorrichtung aus Fig. 1 weist ein Ein-/Ausgabe-Subsystem (I/O) mit drei Eingangskanälen 1, 2, 3 auf.

Über den ersten Eingangskanal 1 wird fortlaufend ein dem Aortendruck des Patienten zumindest näherungsweise entsprechendes Drucksignal eingelesen. Es kann sich dabei um ein analoges Sensor-Signal handeln, welches mittels eines Analog-Digital-Wandlers digitalisiert wird, oder aber es wird bereits ein digitales Signal von einem externen Druckmeßumformer 4 eingelesen.

In der Praxis dient als dem Aortendruck näherungsweise entsprechender Druck ein vorteilhafterweise möglichst aortennah über einen arteriellen Katheter 5 gemessener arterieller Druck. Als Meßort kann eine Beinarterie 6 dienen, wie angedeutet im skizzierten Ausschnitt des Gefäßsystems mit Herz 7, Aortenbogen 8, Lungenkreislauf 9 und Körperkreislauf 10.

Der arterielle Katheter 5 enthält ferner einen Temperatursensor 11, welcher für eine Thermodilutionsmessung zur Kalibrierung eingesetzt werden kann. Das digitale Meßsignal des zugehörigen Temperaturmeßumformers 12 wird über den zweiten Eingangskanal 2 eingelesen. Selbstredend kann jedoch auch das Temperatursignal als Analogsignal eingelesen und mittels eines Analog-Digital-Wandlers digitalisiert werden.

Über den dritten Eingangskanal 3 wird ein den zentralvenösen Druck CVP des Patienten zumindest näherungsweise entsprechendes Drucksignal eingelesen. Auch dieses Signal kann analog oder digital über einen weiteren Druckmeßumformer 13 eingelesen werden. Ein geeigneter Meßort ist die obere Hohlvene 15 des Patienten. Alternativ kann jedoch der zentralvenöse Druck CVP des Patienten auch abgeschätzt werden; unter Umständen genügt dabei eine Abschätzung als konstanter Wert.

Der eingesetzte zentralvenöse Katheter 16 weist ein weiteres Lumen 17 auf, über welches ein gekühlter Bolus für die Durchführung der transpulmonalen Thermodilutionsmessung zur Kalibrierung injiziert werden kann. Im Rahmen der Kalibrierung werden, wie oben erläutert, die Kalibrierwerte SVRcal, τcal und Pdcal für den systemischen Widerstand, die Zeitkonstante und den diastolischen arteriellen Druck ermittelt.

Das Ein-/Ausgabe-Subsystem (I/O) kann einen oder mehrere Ausgabe- bzw. Steuerkanäle 14 aufweisen, welche beispielsweise dem Zusammenwirken mit Peripheriegeräten oder ähnlichem dienen.

Die der Signalverarbeitung dienenden Komponenten der Vorrichtung sind über einen zentralen Bus (BUS) miteinander verbunden.

Das eingelesene Drucksignal wird als Funktion der Zeit p(t) im Arbeitsspeicher (RAM) zwischengespeichert. Die Funktion p(t) wird von der zentralen Recheneinheit (CPU) verarbeitet, um das Herzeitvolumen PCCO und weitere hämodynamische Parameter hieraus zu berechnen. Ein entsprechendes Steuerprogramm, welches die Recheneinheit (CPU) zur Durchführung der entsprechenden Berechnungsschritte veranlaßt, ist in einem Festspeicher (ROM) abgelegt.

Die Berechnung umfaßt dabei folgende Schritte:

Der Übergang zwischen Systole und Diastole wird wie oben beschrieben als Ort der maximalen Krümmung der Druckkurve bestimmt.

Die Zeitkonstante τ wird aus der Druckkurve ermittelt und der systemische Gefäßwiderstand SVRk für die aktuelle, k-te Pulsperiode gemäß der Beziehung

$$SVRk = SVRcal \cdot (\tau / \tau cal)^a \cdot (Pd / Pdcal)^b$$

berechnet.

Aus den Druckwerten einer Pulsperiode bzw. den Druckwerten der Systole einer Pulsperiode wird numerisch das Schlagvolumen SVk der aktuellen Pulsperiode gemäß folgender Beziehung (mit Zählvariable i) ermittelt:

$$SV_k \propto \sum \left( \frac{p_i(t)}{SVR_k} + C_k(p_i) \frac{dp_i}{dt} \right)$$

worin

$$C_k(p) = (MAP\text{-}CVP)k / [SVRk \cdot <dp/dt>k] \cdot f(MAP, pi)$$

eingesetzt wird. Die Differenz zwischen mittlerem arteriellen Druck MAP und zentralvenösem Druck CVP wie auch die mittlere Steigung der Druckkurve in der Diastole <dp/dt> sind für die aktuelle Pulsperiode neu bestimmt. Die Korrekturfunktion f(MAP,pi) wird berechnet wie oben beschrieben.

Das in der aktuellen (k-ten) Pulsperiode errechnete Herzzeitvolumen ergibt sich dann als

$$PCCOk = SVk \cdot HR$$

Das Steuerprogramm im Festspeicher (ROM) kann selbstredend weitere Routinen enthalten, welche der Vorrichtung zusätzliche Funktionalitäten verleihen.

Über ein Display-Subsystem 18 können die Funktion p(t) dargestellt und das Herzeitvolumen PCCO sowie ggf. weitere hämodynamische Parameter ausgegeben werden.

Selbstredend kann die Vorrichtung mit weiteren dem Fachmann an sich bekannten Komponenten ausgestattet sein, beispielsweise Massenspeichermedien zur Aufzeichnung von Rohdaten und/oder berechneten hämodynamischen Parametern. Die Recheneinheit (CPU) kann mit einem oder mehreren herkömmlichen Mikroprozessoren, ggf. unterstützt durch Coprozessoren zur Beschleunigung von Fließkommaoperationen, aber auch mit sogn. digitalen Signalprozessoren (DSP) ausgestattet sein. Entsprechende Lösungen, wie auch weitere Details der Hardware-Ausführung, können analog üblicher Pulskonturanalyse-Geräte gemäß dem Stand der Technik umgesetzt sein.

**Patentansprüche**

1. Vorrichtung zur Ermittlung eines hämodynamischen Parameters eines Patienten mittels Pulskonturanalyse, aufweisend

   einen Eingangskanal (1) zum Einlesen eines dem Aortendruck oder einem aortennahen arteriellen Druck eines Patienten zumindest näherungsweise entsprechenden, zeitlich veränderlichen Drucksignals als Funktion der Zeit p(t), und

   eine Auswerteeinheit zur Berechnung des hämodynamischen Parameters unter Verwendung der Funktion p(t) und des systemischen Widerstands des Körpers des Patienten SVR,

   wobei die Auswerteinheit zur regelmäßigen Neuberechnung des systemischen Widerstands des Körpers des Patienten SVR unter Verwendung der Funktion p(t) ausgestattet ist,

   **dadurch gekennzeichnet, dass** die Neuberechnung des systemischen Widerstands SVR die Bestimmung der Zeitkonstante $\tau$ des näherungsweise exponentiellen Druckabfalls während der Diastole umfasst.

2. Vorrichtung gemäß Anspruch 1, welche Speichermittel (RAM) zum Zwischenspeichern des eingelesenen Drucksignals zumindest über den Pulszyklus als Funktion der Zeit P(t) aufweist.

3. Vorrichtung gemäß Anspruch 1, wobei die Neuberechnung des systemischen Widerstands als Produkt

$$SVRcal \cdot (\tau / \tau cal)^a \cdot (Pd / Pdcal)^b$$

   vorgesehen ist, worin
   SVRcal ein Kalibrierwert des systemischen Widerstands,
   $\tau$cal ein Kalibrierwert der Zeitkonstante $\tau$,
   Pd der diastolische arterielle Druck,
   Pdcal ein Kalibrierwert des diastolischen arterielle Drucks; und
   a, b empirisch bestimmte oder abgeschätzte Exponenten sind.

4. Vorrichtung gemäß Anspruch 3, wobei die Kalibrierwerte SVRcal, $\tau$cal und APdcal im Rahmen einer Kalibriermessung mittels Thermodilution bestimmt sind.

5. Vorrichtung gemäß einem der Ansprüche 3-4, wobei $0,15 < a < 0,6$, vorzugsweise $a=0,3$ ist.

6. Vorrichtung gemäß einem der Ansprüche 3-5, wobei $0,5 < b < 2$, vorzugsweise $b=1$ ist.

7. Vorrichtung gemäß einem der vorangehenden Ansprüche, wobei die Auswerteinheit ferner zur regelmäßigen Neuberechnung von Parametern der Compliance-Funktion C(p) unter Verwendung der Funktion p(t) ausgestattet ist.

8. Vorrichtung gemäß Anspruch 7, wobei die Neuberechnung der Compliance-Funktion C(p) vorgesehen ist nach der Formel

$$C(p) = (MAP-CVP)k/[SVR \cdot <dp/dt>k] \cdot f(p)$$

worin

(MAP-CVP)k die Differenz aus mittlerem arteriellem Druck und zentralvenösem Druck der Diastole der aktuellen Pulsperiode,

<dp/dt>k die mittlere Steigung der arteriellen Druckkurve in der Diastole der aktuellen Pulsperiode, und

f(p) eine Korrekturfunktion ist.

9. Vorrichtung gemäß Anspruch 8 wobei die Korrekturfunktion die Form

$$f(p) = 1 / [k1 \cdot p / MAP - k2 - k3 \cdot (p / MAP)^2]$$

mit empirirsch bestimmten oder abgeschätzten Koeffizienten k1, k2, k3 besitzt.

10. Vorrichtung gemäß Anspruch 9, wobei k1=9/5, k2=17/25 und k3=9/2 ist.

11. Vorrichtung gemäß einem der vorangehenden Ansprüche, wobei der hämodynamische Parameter das Herzzeitvolumen CO als Produkt der Pulsrate HR und des Schlagvolumens SV ist.

12. Vorrichtung gemäß Anspruch 11. wobei die Berechnung des Schlagvolumens als Integral

$$\int \{p(t)/SVR + C(p) \cdot dp/dt\} \, dt$$

vorgesehen ist, worin SVR der systemische Widerstand ist.

13. Vorrichtung gemäß einem der vorangehenden Ansprüche, wobei die Auswerteeinheit Differenziermittel zum Bilden der zweiten Ableitung y'' aus der Funktion p(t), sowie Auswertemittel zur Ermittlung eines Orts maximaler Krümmung der Funktion p(t) in einem Ermittlungsbereich zwischen dem maximalen und dem minimalen Funktionswert des Pulszyklus als Ort des Übergangs zwischen Systole und Diastole aufweist.

14. Vorrichtung gemäß einem der vorangehenden Ansprüche, aufweisend Ausgabemittel (18) zum Ausgeben des hämodynamischen Parameters.

15. Vorrichtung gemäß einem der vorangehenden Ansprüche, aufweisend Anschlussmittel zum Anschluss eines für die Druckmessung geeigneten arteriellen Katheters (5).

16. Vorrichtung gemäß einem der vorangehenden Ansprüche, aufweisend einen Eingangskanal (3) zum Einlesen eines dem zentralvenösen Druck des Patienten zumindest näherungsweise entsprechenden, zeitlich veränderlichen Drucksignals.

17. Vorrichtung gemäß Anspruch 16, ferner aufweisend Anschlussmittel zum Anschluss eines für die Druckmessung geeigneten zentralvenösen Katheters (16).

## Claims

1. Device for determining a haemodynamic parameter of a patient by means of pulse contour analysis, having an input channel (1) for reading in a pressure signal that varies over time and at least approximately corresponds to an aortic pressure or an arterial pressure close to the aorta of a patient, as a function of the time p(t), and an evaluation unit for calculating the haemodynamic parameter using the function p(t) and the systemic resistance of the body of the patient SVR, wherein the evaluation unit is equipped to regularly recalculate the systemic resistance of the body of the patient SVR using the function p(t), **characterised in that** the recalculation of the systemic resistance SVR comprises determining the time constant τ of the approximately exponential pressure drop during the diastole.

2. Device according to claim 1, which has memory means (RAM) for temporarily storing the pressure signal read in, at least over the pulse cycle, as a function of the time P(t).

3. Device according to claim 1, wherein the recalculation of the systemic resistance is provided as the product

$$SVRcal \cdot (\tau / \tau cal)^{a} \cdot (Pd / Pdcal)^{b}$$

wherein,
SVRcal is a calibration value of the systemic resistance,
$\tau$ cal is a calibration value of the time constant $\tau$,
Pd is the diastolic arterial pressure,
Pdcal is a calibration value of the diastolic arterial pressure, and
a, b are empirically determined or estimated exponents.

4. Device according to claim 3, wherein the calibration values SVRcal, $\tau$ cal and APdcal are determined in the course of a calibration measurement by means of thermodilution.

5. Device according to either of claims 3-4, wherein 0.15 < a < 0.6, preferably a = 0.3.

6. Device according to any one of claims 3-5, wherein 0.5 < b < 2, preferably b = 1.

7. Device according to any one of the preceding claims, wherein the evaluation unit is furthermore equipped to regularly recalculate parameters of the compliance function C(p) using the function p(t).

8. Device according to claim 7, wherein the recalculation of the compliance function C(p) is provided according to the formula

$$C(p) = (MAP - CVP) k / [SVR < dp/dt > k] \cdot f(p)$$

wherein,
(MAP - CVP) k is the difference between the mean arterial pressure and central venous pressure of the diastole of the current pulse period,
< dp/dt > k is the mean gradient of an arterial pressure curve in the diastole of the current pulse period, and
f(p) is a correction function.

9. Device according to claim 8, wherein the correction function has the form

$$f(p) = 1 / [k1 \cdot p / MAP - k2 - k3 \cdot (p / MAP)^{2}]$$

with empirically determined or estimated coefficients k1, k2, k3.

10. Device according to claim 9, wherein k1 = 9/5, k2 = 17/25 and k3 = 9/2.

11. Device according to any one of the preceding claims, wherein the haemodynamic parameter is the cardiac output CO as a product of the pulse rate HR and of the stroke volume SV.

12. Device according to claim 11, wherein the calculation of the stroke volume is provided as the integral

$$\int \{p(t) / SVR + C(p) \cdot dp/dt\} dt$$

wherein SVR is the systemic resistance.

13. Device according to any one of the preceding claims, wherein the evaluation unit has differentiation means to form the second derivative y" from the function p(t), as well as evaluation means to determine a site of maximum curvature of the function p(t) in a determination region between the maximum and the minimum function value of the pulse cycle as the site of the transition between the systole and diastole.

14. Device according to any one of the preceding claims, having output means (18) to output the haemodynamic parameter.

15. Device according to any one of the preceding claims, having connection means to connect an arterial catheter (5) suitable for the pressure measurement.

16. Device according to any one of the preceding claims, having an input channel (3) to read in a pressure signal that varies over time and at least approximately corresponds to the central venous pressure of the patient.

17. Device according to claim 16, furthermore having connection means to connect a central venous catheter (16) suitable for the pressure measurement.


**Revendications**

1. Dispositif pour déterminer un paramètre hémodynamique d'un patient par analyse du contour du pouls, présentant un canal d'entrée (1) pour la mise en mémoire d'un signal de pression qui change au cours du temps, correspondant au moins de manière approchée à la pression aortique ou à une pression artérielle proche de l'aorte d'un patient, en fonction du temps p(t), et
une unité d'évaluation pour le calcul du paramètre hémodynamique avec utilisation de la fonction p(t) et de la résistance systémique du corps du patient SVR,
l'unité d'évaluation étant équipée pour un recalcul régulier de la résistance systémique du corps du patient SVR avec utilisation de la fonction p(t),
**caractérisé par le fait que** le recalcul de la résistance systémique SVR comprend la détermination de la constante de temps τ de la chute de pression exponentielle de manière approchée pendant la diastole.

2. Dispositif selon la revendication 1, lequel présente des moyens de mémoire (RAM) pour la mise en mémoire intermédiaire du signal de pression mis en mémoire au moins sur le cycle du pouls en fonction du temps P(t).

3. Dispositif selon la revendication 1, suivant lequel le recalcul de la résistance systémique est fourni en tant que produit :

$$\text{SVRcal.}(\tau/\tau\text{cal})^a.(\text{Pd}/\text{Pdcal})^b$$

où :

SVRcal est une valeur d'étalonnage de la résistance systémique ;
τcal est une valeur d'étalonnage de la constante de temps τ ;
Pd est la pression artérielle diastolique ;
Pdcal est une valeur d'étalonnage de la pression artérielle diastolique ; et
a, b sont des exposants déterminés ou estimés de façon empirique.

4. Dispositif selon la revendication 3, suivant lequel les valeurs d'étalonnage SVRcal, τcal et APdcal sont définies dans le cadre d'une mesure d'étalonnage par thermodilution.

5. Dispositif selon l'une des revendications 3-4, suivant lequel $0,15 < a < 0,6$, de préférence $a = 0,3$.

6. Dispositif selon l'une des revendications 3-5, suivant lequel $0,5 < b < 2$, de préférence $b = 1$.

7. Dispositif selon l'une des revendications précédentes, suivant lequel l'unité d'évaluation est équipée en outre pour le recalcul régulier de paramètres de la fonction de compliance C(p) avec utilisation de la fonction p(t).

8. Dispositif selon la revendication 7, suivant lequel le recalcul de la fonction de compliance C(p) est fourni d'après la

formule :

$$C(p) = (MAP.CVP)k/[SVR.<dp/dt>k].f(p)$$

où :

(MAP.CVP)k est la différence entre la pression artérielle moyenne et la pression veineuse centrale de la diastole de la période du pouls courante ;
<dp/dt>k est la pente moyenne de la courbe de pression artérielle dans la diastole de la période du pouls courante, et
f(p) est une fonction de correction.

9. Dispositif selon la revendication 8, suivant lequel la fonction de correction possède la forme :

$$f(p) = 1/[k1.p/MAP-k2-k3.(p/MAP)^2]$$

avec des coefficients k1, k2, k3 déterminés ou estimés de manière empirique.

10. Dispositif selon la revendication 9, suivant lequel k1 = 9/5, k2 = 17/25 et k3 = 9/2.

11. Dispositif selon l'une des revendications précédentes, suivant lequel le paramètre hémodynamique est le débit cardiaque CO comme produit de la fréquence du pouls HR et du volume d'éjection systolique SV.

12. Dispositif selon la revendication 11, suivant lequel le calcul du volume d'éjection systolique est prévu comme intégrale

$$\int \{p(t)/SVR + C(p).dp/dt\}dt,$$

où SVR est la résistance systémique.

13. Dispositif selon l'une des revendications précédentes, suivant lequel l'unité d'évaluation présente des moyens de dérivation pour la formation de la dérivée seconde y'' à partir de la fonction p(t), ainsi que des moyens d'évaluation pour la détermination d'un lieu de courbure maximale de la fonction p(t) dans une plage de détermination entre la valeur de fonction maximale et la valeur de fonction minimale du cycle du pouls comme lieu de la transition entre systole et diastole.

14. Dispositif selon l'une des revendications précédentes, présentant des moyens de sortie (18) pour la sortie du paramètre hémodynamique.

15. Dispositif selon l'une des revendications précédentes, présentant des moyens de raccordement pour le raccordement d'un cathéter artériel (5) approprié pour la mesure de la pression.

16. Dispositif selon l'une des revendications précédentes, présentant un canal d'entrée (3) pour la mise en mémoire d'un signal de pression qui change au cours du temps correspondant au moins de manière approchée à la pression veineuse centrale du patient.

17. Dispositif selon la revendication 16, présentant en outre des moyens de raccordement pour le raccordement d'un cathéter veineux central (16) approprié pour la mesure de pression.

*Fig. 1*

**EP 1 598 005 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19814371 A1

- EP 1236435 A1